(19) Europäisches Patentamt
European Patent Office
Office européen des brevets

(11) Numéro de publication: **0 122 206**
**A2**

(12) # DEMANDE DE BREVET EUROPEEN

(21) Numéro de dépôt: **84420049.3**

(22) Date de dépôt: **15.03.84**

(51) Int. Cl.³: **C 07 D 471/04**, A 01 N 43/90 // (C07D471/04, 235/00, 221/00)

(30) Priorité: **16.03.83 FR 8304531**

(43) Date de publication de la demande: **17.10.84 Bulletin 84/42**

(84) Etats contractants désignés: **AT BE CH DE FR GB IT LI LU NL SE**

(71) Demandeur: **RHONE-POULENC AGROCHIMIE, 14-20, rue Pierre Baizet, F-69009 Lyon (FR)**

(72) Inventeur: **Giraudon, Raymond, 5 Rue des Colverts, F-77330 Lesigny (FR)**
Inventeur: **Santini, Georges, 7 Rue Jeanne d'Arc, F-94320 Thiais (FR)**

(74) Mandataire: **Chaumette, Michel et al, RHONE-POULENC AGROCHIMIE BP 9163-09, F-69263 Lyon Cedex 1 (FR)**

(54) **Nouveaux dérivés de cyano-2 imidazopyridine, leur préparation et leur utilisation comme fongicides.**

(57) L'invention concerne de nouveaux dérivés de la cyano-2 imidazopyridine.

Ces composés répondent à la formule (I):

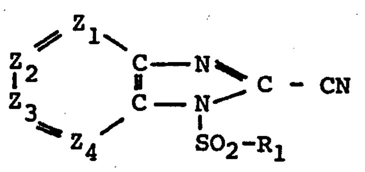

(I)

dans laquelle:

— $R_1$ représente un radical alkyle ou cycloalkyle inférieur éventuellement halogéné ou un radical amino, éventuellement substitué par un ou deux radicaux alkyle inférieur,

— l'un des groupes $Z_1$, $Z_2$, $Z_3$ et $Z_4$ représente l'atome d'azote et les autres sont choisis indépendamment parmi le groupe CH et les groupes CR dans lesquels R représente un atome d'halogène ou un radical alkyle inférieur éventuellement substitué, alcoxy inférieur éventuellement substitué, alkylthio inférieur éventuellement substitué, nitro ou cyano.

Ils sont utilisables en agriculture pour la lutte contre les champignons phytopathogènes.

EP 0 122 206 A2

La présente demande de brevet concerne de nouveaux dérivés de cyano-2 imidazopyridine ainsi que la préparation de ces composés. Elle concerne de plus les compositions pesticides, notamment antifongiques, contenant comme matière active au moins un de ces composés, ainsi que les traitements antifongiques effectués au moyen de ces nouveaux composés.

Elle concerne enfin, en tant que produits nouveaux, certains des composés utilisés pour la mise en oeuvre du procédé de préparation mentionné plus haut.

Les nouveaux dérivés de cyano-2 imidazopyridine selon l'invention sont caractérisés en ce qu'ils répondent à la formule générale (I) :

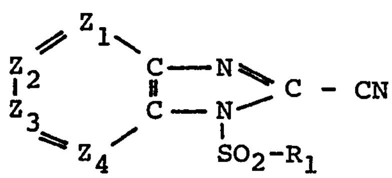

(I)

dans laquelle :

-$R_1$ représente un radical alkyle ou cycloalkyle inférieur éventuellement substitué par un ou plusieurs atomes d'halogène (tel que par exemple les radicaux méthyle, éthyle, isopropyle, trichlorométhyle...etc ) ; ou un radical amino, éventuellement substitué par un ou deux radicaux alkyle inférieur, identiques ou différents, (par exemple les radicaux diméthylamino ou diéthylamino, ou méthyléthylamino, etc.)

- l'un des groupes $Z_1$, $Z_2$, $Z_3$ et $Z_4$ représente l'atome d'azote et les autres sont choisis indépendemment parmi le groupe CH et les groupes CR dans lesquels R représente un atome d'halogène ou un radical nitro ou cyano ou un radical alkyle inférieur, alkylthio inférieur ou alkoxy inférieur, ces radicaux alkyle, alkylthio et alkoxy étant éventuellement substitués, par exemple par un ou plusieurs atomes d'halogènes, étant entendu que

les substituants R peuvent être soit identiques, soit différents.

Au sens du présent texte, l'adjectif "inférieur", appliqué à un radical organique signifie que ce radical comporte au plus six atomes de carbone.

Parmi les composés selon la formule (I), la présente demande de brevet concerne plus particulièrement les composés pour lesquels :

$R_1$ a la même signification que dans la formule (I), et représente de préférence un radical dialkylamino comportant de 2 à 4 atomes de carbone

les groupes $Z_1$, $Z_2$, $Z_3$ et $Z_4$ ont respectivement la signification suivante :

- le groupe $Z_1$ ou le groupe $Z_4$ représente l'atome d'azote,
- de un à trois des autres groupes (c'est-à-dire de un à trois des groupes $Z_2$, $Z_3$ et $Z_4$ lorsque $Z_1$ représente l'atome d'azote, ou de un à trois des groupes $Z_1$, $Z_2$ et $Z_3$ lorsque $Z_4$ représente l'atome d'azote) représentent le groupe CH,
- et les groupes restants représentent le groupe CR dans lequel R a la même signification que dans la formule (I).

De préférence R représente un atome d'halogène ou un radical alkyle comportant de 1 à 3 atomes de carbone et éventuellement substitué par un ou plusieurs atomes d'halogènes (tel que le radical trifluorométhyle) ou le radical nitro ou le radical cyano.

Selon que $Z_4$ ou $Z_1$ représente l'atome d'azote, ces composés qui constituent un objet particulier de l'invention correspondent respectivement soit à la formule (I bis) soit à la formule (I ter) ci-après :

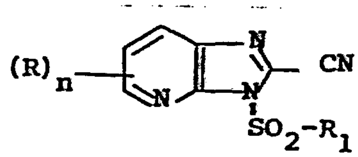

(I bis)

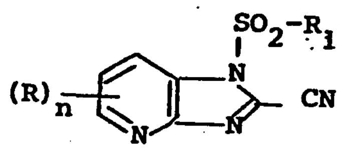

(I ter)

dans lesquelles R et $R_1$ ont la même signification que dans la formule (I) et n est égal à 0, 1 ou 2 étant entendu que lorsque n est égal à 2 les substituants R peuvent être identiques ou différents.

De préférence, les substituants présents dans les formules (I bis) et (I ter) ont la signification ci-après :

- $R_1$ représente un radical dialkylamino comportant de 2 à 4 atomes de carbone,
- n est égal à 0, 1 ou 2,
- et R représente un atome d'halogène ou un radical alkyle comportant de 1 à 3 atomes de carbone, éventuellement substitué par un ou plusieurs atomes d'halogènes, ou le radical nitro, ou le radical cyano,
- étant entendu que lorsque n est égal à 2, les substituants R peuvent être soit identiques soit différents.

Les composés selon la formule (I) présentent d'une façon générale une excellente activité antifongique vis-à-vis de différentes familles de champignons phytopathogènes, notamment vis-à-vis des phycomycètes tels que les mildious de la pomme de terre et de la tomate (Phytophthora infestans), le mildiou du tabac (Peronospora tabacina) et le mildiou de la vigne (Plasmopara viticola). Ces propriétés seront décrites plus en détail dans les exemples biologiques illustrant la présente demande de brevet.

Certains dérivés d'imidazopyridines ont déjà été décrits dans la littérature. Ainsi, le brevet du Royaume-Uni n° 1213654 décrit une très vaste famille d'imidazopyridines substitués sur l'atome de carbone en position 2 par un radical trifluorométhyle ou pentafluoroéthyle et il signale que ces composés sont

utilisables principalement comme herbicides, qu'ils trouvent également utilisation comme insecticides, mollusquicides, ou fongicides, et que certains d'entre eux montrent de plus une activité contre les mouches, les moustiques et les araignées.

Les composés revendiqués dans la présente demande de brevet sont distincts de ceux décrits dans ce brevet n° 1213654. Leurs excellentes propriétés antifongiques n'étaient pas évidentes compte-tenu de cet art antérieur.

L'invention concerne de plus un procédé pour préparer les composés selon la formule (I).

Ce procédé est caractérisé en ce qu'il consiste à faire réagir la cyano-2 imidazopyridine de formule (II)

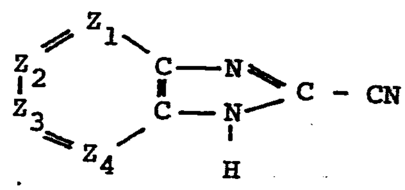

(II)

dans laquelle $Z_1$, $Z_2$, $Z_3$ et $Z_4$, ont la même signification que dans la formule (I), ou un sel de ce composé (II), avantageusement un sel de métal alcalin, ou alcalino-terreux, ou d'ammonium éventuellement substitué, sur un halogénure de formule (III)

$$X\text{-}SO_2R_1 \qquad (III)$$

dans laquelle $R_1$ a la même signification que dans la formule (I) et X représente un atome d'halogène, de préférence un atome de chlore.

La réaction de la cyano-2 imidazopyridine (II) sur l'halogènure (III) s'effectue avantageusement en présence d'un accepteur d'acide, en milieu anhydre ou non anhydre, dans un solvant inerte dans les conditions de la réaction, généralement à la température d'ébullition du solvant.

Comme accepteurs d'acide, on peut mentionner des bases minérales comme par exemple la soude ou la potasse, les carbonates de métaux alcalins ou de métaux alcalino-terreux, des bases azotées comme la triéthylamine. Comme solvants on utilise avantageusement des solvants aprotiques polaires comme par exemple le diméthylformamide, le diméthylacétamide, le diméthylsulfoxyde, l'acétone, la méthyl-éthyl-cétone, l'acétonitrile, la N-méthylpyrrolidone. Si désiré, cette réaction peut être effectuée en présence d'un catalyseur approprié. Comme catalyseur utilisable, on peut mentionner des catalyseurs de transfert de phases, tels que, par exemple, des dérivés d'ammonium quaternaire.

La réaction du sel (de métal alcalin ou alcalino-terreux ou d'ammonium éventuellement substitué) de la cyano-2 imidazopyridine (II) avec l'halogénure (III) ne nécessite pas la présence d'un accepteur d'acide. Elle s'effectue avantageusement dans un solvant aprotique polaire, inerte dans les conditions de la réaction, anhydre ou non, généralement à la température d'ébullition du solvant. Les solvants aprotiques polaires cités plus haut peuvent être utilisés dans cette réaction.

Si désiré, cette réaction peut aussi être effectuée en présence d'un catalyseur approprié, tel que, par exemple, un catalyseur de transfert de phases (par exemple un dérivé d'ammonium quaternaire).

Le sel de métal alcalin ou alcalino-terreux ou d'ammonium du composé (II) est préparé dans une opération préalable, éventuellement effectuée in situ, par action d'une base appropriée (par exemple soude, potasse, ammoniaque) ou d'un carbonate de métal alcalin, ou d'un alcoolate de métal alcalin (par exemple méthylate de sodium ou éthylate de sodium ou potassium) sur ce composé (II).

En fin de réaction, quelque soit le procédé utilisé, le composé formé est isolé du milieu réactionnel

par tout moyen en soi connu tel que, par exemple par distillation du solvant, ou par cristallisation du produit dans le milieu réactionnel, ou par filtration, puis, si nécessaire, ce composé est purifié par des méthodes usuelles telles que recristallisation dans un solvant approprié ou chromatographie liquide sur colonne de silice ou alumine.

La cyano-2 imidazopyridine, de formule (II), servant de produit de départ dans le procédé de préparation selon l'invention, peut être préparée par action de l'ammoniaque, à une température de l'ordre de 10°C, sur la trichlorométhyl-2 imidazopyridine de formule (IV) :

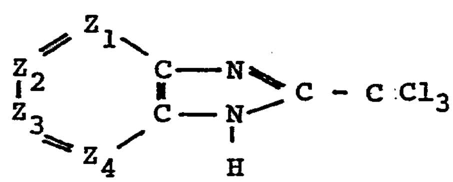

(IV)

dans laquelle $Z_1$, $Z_2$, $Z_3$ et $Z_4$, ont la même signification que dans la formule (I).

La trichlorométhyl-2 imidazopyridine de formule (III) est elle même obtenue par action du trichloroacétimidate de méthyle, de formule (V)

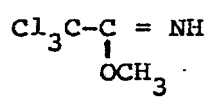

(V)

en présence d'acide acétique sur la diamino -2,3 pyridine de formule (VI) :

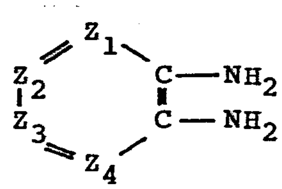

(VI)

dans laquelle $Z_1$, $Z_2$, $Z_3$ et $Z_4$, ont la même signification que dans la formule (I).

Les composés répondant aux formules (II) et (IV) sont compris dans le cadre de la présente invention, en tant que produits chimiques nouveaux, utilisables notamment pour la mise en oeuvre du procédé de préparation décrit plus haut.

Ils répondent à la formule générale (VII)

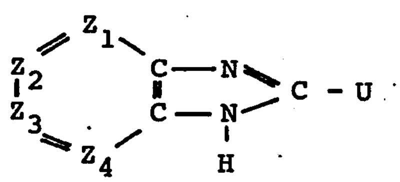

(VII)

dans laquelle $Z_1$, $Z_2$, $Z_3$ et $Z_4$, ont la même signification que dans la formule I et U représente soit le radical cyano soit le radical trichlorométhyle.

Les exemples ci-après, illustrent l'invention sans toutefois la limiter.

La structure des composés décrits dans ces exemples a été confirmée par spectrométrie de résonance magnétique nucléaire (RMN) et/ou par spectrométrie infrarouge.

Exemple 1 :

Préparation de la 3 H-cyano-2 diméthylsulfamoyl-3 imidazo [4,5-b] pyridine de formule :

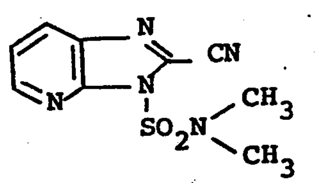

(n°1A)

et de la 1 H-cyano-2 diméthylsulfamoyl-1 imidazo [4,5-b] pyridine de formule :

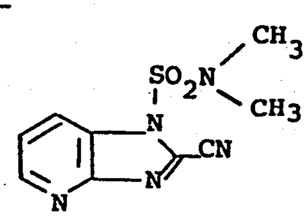

(n°1B)

Dans une solution de 8 g de cyano-2 imidazo [4,5-b] pyridine dans 100 ml d'acétonitrile, on ajoute 8,4 g de carbonate de potassium anhydre.

Le mélange obtenu est chauffé, à la température de reflux de l'acétonitrile, pendant une heure puis refroidi à 30°C et on lui ajoute alors, en une seule fois 8,25 g (6,15 ml) de chlorure de diméthylsulfamoyle.

Le mélange réactionnel est alors chauffé à la température de reflux de l'acétonitrile puis maintenu à cette température pendant une heure.

Le mélange réactionnel est ensuite chauffé, puis refroidi, ensuite filtré, et enfin concentré à sec sous pression réduite (20 millibars environ).

Le résidu solide obtenu (13,5 g) est repris par 150 ml d'acétate d'éthyle puis chromatographié sur colonne de silice.

On obtient ainsi 2,9 g du composé n°1A, sous la forme de cristaux blancs, fondant à 166°C et 5,6 g du composé n°1B, sous la forme de cristaux blancs fondant à 150°C.

La cyano-2 imidazo [4,5-b] pyridine, dont la formule est la suivante :

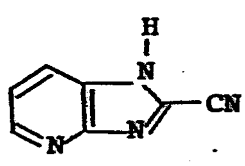

a été préparée selon le procédé décrit ci-après :

Dans 250 ml d'ammoniaque à 34 %, agités vigoureusement et maintenus à 10°C environ, on charge, par petites fractions, 21,5 g de trichlorométhyl-2 imidazo [4,5-b] pyridine. Après la fin de l'addition, on agite le mélange réactionnel pendant environ une heure en laissant remonter la température jusqu'à la température anbiante (20 à 25°C). On filtre le mélange réactionnel sur silice

supercel et on acidifie le filtrat jusqu'à pH = 6, par 200 ml d'acide chlorhydrique de densité 1,19, en maintenant la température en dessous de 10°C. Par filtration du précipité puis extraction par l'acétate d'éthyle des eaux-mères et concentration, on obtient au total 8 g de cyano-2 imidazo [4,5-b] pyridine, fondant à 200°C environ. Pureté environ 80 %

La tricnlorométhyl-2 imidazo [4,5-b] pyridine, de formule :

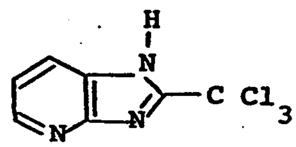

a été préparée selon le procédé décrit ci-après :

Dans une solution de 19,7 g de diamino-2,3 pyridine dans 400 ml d'acide acétique, on ajoute en une seule fois 32,8 g (22,75 ml) de trichloro acétimidate de méthyle et on laisse reposer le mélange réactionnel à la température ambiante (20 à 25°C) pendant 72 heures.

On coule alors le mélange réactionnel obtenu sur 1,5 litre d'eau glacée. Le précipité cristallisé est essoré, lavé à l'eau et sèché. On obtient ainsi 21,15 g de trichlorométhyl-2 imidazo [4,5-b] pyridine fondant à 210°C.

Exemple 2

En opérant selon la méthode décrite à l'exemple précédent, à partir des matières de départ appropriées, les composés et mélanges ci-après ont été préparés :

n°2A : 3 H-chloro-6 cyano-2 diméthylsulfamoyl-3 imidazo [4,5-b] pyridine,

n°2B : 1 H-cnloro-6 cyano-2 diméthylsulfamoyl-1 imidazo [4,5-b] pyridine,

n°3A : 3 H-cyano-2 dicnloro-5,6 diméthylsulfamoyl-3 imidazo [4,5-b] pyridine, en mélange (65/35) avec composé 3B

n°3B : 1 H-cyano-2 dichloro-5,6 diméthylsulfamoyl-1 imidazo [4,5-b] pyridine,

n°4A : 3 H-cyano-2 diméthylsulfamoyl-3 méthyl-7 imidazo [4,5-b] pyridine,
n°5A : 3 H-bromo-6 cyano-2 diméthylsulfamoyl-3 imidazo [4,5-b] pyridine,
n°5B : 1 H-bromo-6 cyano-2 diméthylsulfamoyl-1 imidazo [4,5-b] pyridine,
n°6A : 3 H-cyano-2 diméthylsulfamoyl-3 méthyl-6 imidazo [4,5-b] pyridine,
n°6B : 1 H-cyano-2 diméthylsulfamoyl-1 méthyl-6 imidazo [4,5-b] pyridine,
Les formules de ces composés ainsi que leurs points de fusion sont décrits dans le tableau I, à la fin de la description.

Exemple 3 :

Tests en serre sur mildiou de la tomate

Des plants de tomate (Lycopersicum esculentum), de variété Marmande, sont cultivés dans des godets. Lorsque ces plants sont agés d'un mois (stade 5 à 6 feuilles, hauteur 12 à 15 cm), ils sont traités par pulvérisation au moyen d'une suspension ou solution aqueuse de la matière à tester, à la concentration désirée et contenant 0,02% d'un condensat de monoléate de sorbitan et de 20 molécules d'oxyde d'éthylène. Chaque plant de tomate reçoit environ 5ml de la solution ou dispersion. Pour chaque concentration de matière active à tester, le traitement est effectué sur huit plants. Des plants utilisés comme témoins sont traités par une solution ne contenant pas de matière active, mais contenant 0,02% du même condensat de monooléate de sorbitan et d'oxyde d'éthylène.

Après séchage pendant 4 heures, on contamine chaque plant par pulvérisation au moyen d'une suspension aqueuse de spores de Phytophthora infestans, responsable du mildiou de la tomate, à raison d'environ 1ml/plant (soit environ $2.10^5$ spores par plant).

Après cette contamination, les plants de tomate

sont mis en incubation pendant trois jours à 20°C environ en atmosphère saturée d'humidité, puis pendant quatre jours à 17°C environ sous 70% à 80% d'humidité relative.

Sept jours après la contamination, on compare les résultats obtenus dans le cas des plants traités par la matière active à tester à ceux obtenus dans le cas des plants utilisés comme témoins et on détermine la concentration minimale inhibitrice entraînant de 95 à 100% d'inhibition du développement du champignon considéré (CMI 95-100).

Dans ces conditions, on observe que, pour les composés ou mélanges de composés décrits dans les exemples précédents, cette concentration a été respectivement la suivante :

| Composé ou mélange testé | CMI (95-100) Phytophthora infestans en mg/l |
|---|---|
| 1B | 8 |
| 2A | 4 |
| 2B | 8 |
| 3A + 3B (65/35) | 1 |
| 3B | 1 |
| 4A | supérieure à 16 mg/l |
| 5A | 8 |
| 5B | inférieure ou égale à 2 mg/l |

Dans cet essai on a observé des pourcentages d'inhibition du développement du champignon considéré compris entre 20 % et 90 % :

- à la dose de 500 mg/l pour les composés n°1A et 6A,
- à la dose de 8 mg/l pour le composé 6B.

Exemple 4 : Test en serre sur mildiou du tabac

On opère comme à l'exemple précédent si ce n'est que les végétaux sont des plants de tabac (*Nicotiana*

*tabacum*) de variété Samson et que ces plants sont contaminés par des spores de *Peronospora tabaci*, responsable du mildiou du tabac.

Dans ces conditions, on a observé que, pour les composés ou mélanges de composés décrits dans les exemples précédents, les concentrations minimales inhibitrices entraînant de 95 à 100% d'inhibition du champignon considéré (CMI 95-100) sont respectivement les suivantes :

| Composés ou mélange testés | CMI (95-100) Peronospora tabaci en mg/l |
|---|---|
| 1B | 16 |
| 2A | inférieure ou égale à 2 |
| 2B | 4 |
| 5B | 8 |
| 6A | inférieure ou égale à 4 |

Dans cet essai, on a observé des pourcentages d'inhibition du développement du champignon considéré compris entre 20 % et 90 % à la dose de 500 mg/l pour les composés n° 1A, et 6B, à la dose de 8 mg/l pour les composés n° 3B et 5A et pour le mélange 3A + 3B (65/35).

Les exemples n°3 et 4 décrits plus haut illustrent la bonne activité antifongique des composés selon l'invention.

Les composés selon l'invention sont avantageusement utilisés comme antifongiques dans le domaine agricole. Ils présentent une action par contact et par systèmie et peuvent être employés à titre préventif et/ou à titre curatif pour la lutte contre divers champignons phytopathogènes tels que par exemple de nombreux phycomycètes et basidiomycètes.

Pour leur emploi dans la pratique, les composés selon l'invention ne sont généralement pas utilisés seuls. Le plus souvent ils sont utilisés dans des compositions qui

comprennent en général, en plus de la matière active, un support (ou diluant) inerte et/ou un agent tensioactif compatibles avec la matière active.

Ces compositions font également partie de la présente invention. Elles contiennent habituellement de 0,001 à 95% en poids de matière active. Leur teneur en agent tensioactif est en général comprise entre 0% et 20% en poids.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment par la plante traitée. Le support peut être solide (argiles, silicates naturels ou synthétiques, silice, craies, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques ; des sels d'acides lignosulfoniques des sels d'acides phénolsulfoniques ou naphtalènesulfoniques ; des polycondensats d'oxyde d'éthylène sur des alcools gras ou sur des acides gras ou sur des amines grasses ou sur des phénols substitués (notamment des alkylphénols ou des arylphénols ou le styrylphénol) ; des sels d'esters d'acides sulfosucciniques ; des dérivés de la taurine (notamment des alkyltaurates) ; des esters phosphoriques d'alcools ou de phénols polyoxyéthylés. La présence d'au moins un agent tensioactif est généralement indispensable surtout lorsque le support inerte n'est pas soluble dans l'eau, et que l'agent vecteur de l'application est l'eau.

Les compositions utilisées dans l'invention peuvent être sous des formes assez diverses, solides ou liquides.

Comme formes de compositions solides, on peut citer les poudres pour poudrage (à teneur en matières actives pouvant aller jusqu'à 100 %).

Comme formes de compositions liquides ou destinées constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,001 à 20 % de matière active. En plus du solvant, les concentrés émulsionnables peuvent contenir, quand c'est nécessaire, un co-solvant approprié et de 2 à 20 % d'additifs appropriés, comme des stabilisants, des agents tensioactifs, notamment des émulsifiants, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application sur les cultures.

Les suspensions concentrées, également applicables en pulvérisation, sont préparées de manière à obtenir un produit fluide stable ne se déposant pas et elles contiennent habituellement de 10 à 75 % de matières actives, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines

matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95 % de matières actives, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici une composition de poudre mouillable à 50 % :

- matière active ........................ 50 %
- alcool gras éthoxylé (agent mouillant) .. 2,5 %
- styrylphénol éthoxylé (agent dispersant). 5 %
- craie (support inerte) ................. 42,5 %

Un autre exemple de poudre mouillable a la composition suivante :

- matière active ........................ 90 %
- alcool gras éthoxylé (agent mouillant) .. 4 %
- styrylphénol éthoxylé (agent dispersant). 6 %

Un autre exemple de poudre mouillable à 50 % a la composition suivante :

- matière active ........................ 50 %
- mélange de tensio-actifs anioniques et non ioniques (agent mouillant) .......... 2,5 %
- lignosulfonate de sodium neutre (agent dispersant) ...................... 5 %
- argile kaolinique (support inerte) ...... 42,5 %

Pour obtenir ces poudres à pulvériser ou poudres mouillables, on mélange intimement la matière active dans des mélangeurs appropriés avec les substances additionnelles et on broie avec des moulins ou autres

broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

Comme cela a déjà été dit, les dispersions et émulsions aqueuses, par exemples des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huile-dans-l'eau et elles peuvent avoir une consistance épaisse comme celle d'une "mayonnaise".

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Les composés de formule (II) peuvent encore être utilisés sous forme de poudres pour poudrage, on peut ainsi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

L'invention concerne de plus un procédé de traitement des végétaux contre les champignons phytopathogènes.

Ce procédé est caractérisé en ce qu'il consiste à appliquer sur ces végétaux une quantité efficace d'un composé selon l'une des formules (I), I bis et I ter. Par "quantité efficace" on entend une quantité suffisante pour

permettre le contrôle et la destruction des champignons présents sur ces végétaux. Les doses d'utilisation peuvent toutefois varier dans de larges limites selon le champignon à combattre, le type de culture, les conditions climatiques, et selon le composé utilisé.

En pratique des doses allant de 5 g/hl à 100 g/hl correspondant sensiblement à des doses de matière active par hectare de 50 g/ha à 1000 g/ha environ donnent généralement de bons résultats.

TABLEAU I

| N° Composé | FORMULE | Point de fusion (°C) |
|---|---|---|
| 2A | 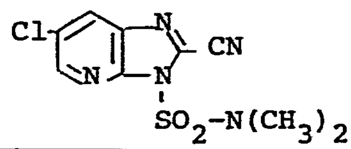 | 196 |
| 2B | 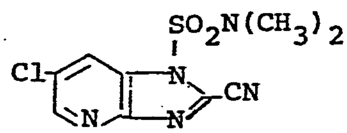 | 172 |
| 3A | 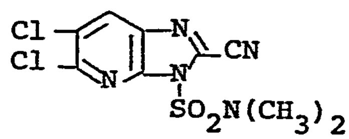 | mélange 3A+3B (65/35) 195-200 |
| 3B | 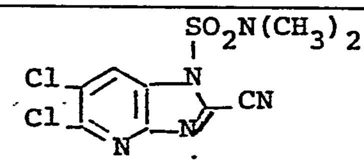 | 215 - 220 (avec décomposition) |
| 4A | 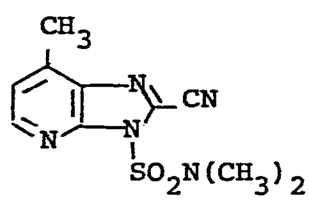 | 180 |
| 5A | 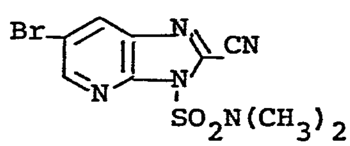 | 172 |
| 5B | 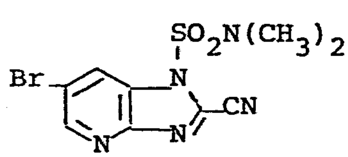 | 210 |

TABLEAU I (suite)

| N° Composé | FORMULE | Point de fusion (°C) |
|---|---|---|
| 6A | 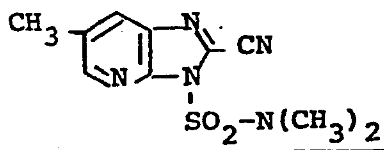 | 190 |
| 6B | 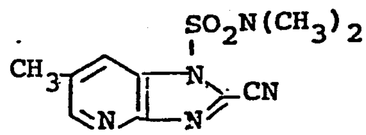 | 190 |

## REVENDICATIONS

1) - Dérivé de cyano-2 imidazopyridine caractérisé en ce qu'il répond à la formule générale (I)

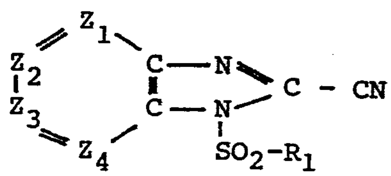

(I)

dans laquelle :
-$R_1$ représente un radical alkyle inférieur éventuellement substitué par un ou plusieurs atomes d'halogène, ou cycloalkyle inférieur éventuellement substitué par un ou plusieurs atomes d'halogène, ou un radical amino, éventuellement substitué par un ou deux radicaux alkyle inférieur, identiques ou différents,
- l'un des groupes $Z_1$, $Z_2$, $Z_3$ et $Z_4$ représente l'atome d'azote et les autres sont choisis indépendemment parmi le groupe CH et les groupes CR dans lesquels R représente un atome d'halogène ou un radical alkyle inférieur éventuellement substitué, alkoxy inférieur éventuellement substitué, alkylthio inférieur éventuellement substitué, nitro ou cyano.

2) - Composé selon la revendication 1 caractérisé en ce qu'il répond à la formule (I bis) :

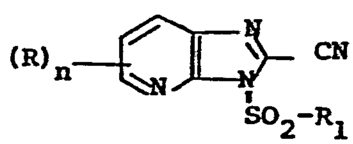

(I bis)

dans laquelle :

- R et $R_1$ ont la même signification que dans la revendication 1 et n est égal à 0, 1 ou 2 étant entendu que lorsque n est égal à 2, les substituants R peuvent être identiques ou différents.

3) - Composé selon la revendication 1 caractérisé en ce qu'il répond à la formule (I ter) :

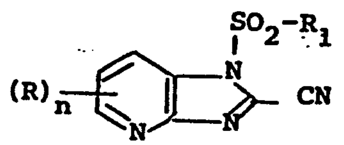

(I ter)

dans laquelle :

- R et $R_1$ ont la même signification que dans la revendication 1 et n est égal à 0, 1 ou 2 étant entendu que lorsque n est égal à 2, les substituants R peuvent être identiques ou différents.

4) - Composé selon l'une des revendications 1 à 3 caractérisé en ce que $R_1$ représente un radical dialkylamino comportant de 2 à 4 atomes de carbone et R représente un atome d'halogène ou un radical alkoxy, alkylthio ou alkyle, éventuellement mono- ou polyhalogéné et comportant de 1 à 3 atomes de carbone, ou le radical nitro ou le radical cyano.

5) - Composition pesticide utilisable notamment pour la lutte contre les champignons phytopathogènes caractérisée en ce qu'elle comprend comme matière active un composé selon l'une des revendications 1 à 4.

6) - Composition selon la revendication 5 caractérisée en ce qu'elle comprend de 0,001 % à 95 % en poids de matière active et de 0 à 20 % en poids d'agent tensio-actif.

7) - Procédé de préparation d'un composé selon la revendication 1, caractérisé en ce qu'il consiste à faire réagir la cyano-2 imidazopyridine de formule (II)

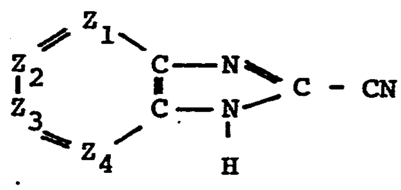

(II)

dans laquelle $Z_1$, $Z_2$, $Z_3$ et $Z_4$, ont la même signification que dans la revendication 1, ou un sel de cette cyano-2 imidazopyridine, sur un halogénure de formule (III) :

$$X - SO_2R_1 \qquad (III)$$

dans laquelle $R_1$ a la même signification que dans la revendication 1 et X représente un atome d'halogène.

8) - Procédé selon la revendication 7 caractérisé en ce que la réaction entre la cyano-2 imidazopyridine et l'halogénure s'effectue dans un solvant aprotique polaire, en présence d'un accepteur d'acide.

9) - Procédé selon la revendication 7 caractérisé en ce que la réaction entre le sel de la cyano-2 imidazopyridine et l'halogénure s'effectue dans un solvant aprotique polaire.

10)- Dérivé de cyano-2 imidazopyridine répondant à la formule (VII) ci-après :

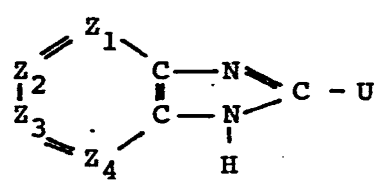

(VII)

dans laquelle $R_1$, $Z_1$, $Z_2$, $Z_3$ et $Z_4$ ont la même signification que dans la revendication 1 et U représente

le radical cyano ou le radical trichlorométhyle.

11)- Procédé de traitement des végétaux contre les champignons phytopathogènes caractérisé en ce qu'il consiste à appliquer sur ces végétaux une quantité efficace d'un composé selon l'une des revendications 1 à 4.

REVENDICATIONS POUR L'AUTRICHE

1) - Composition pesticide à usage agricole, utilisable notamment pour la lutte contre les champignons phytopathogènes, caractérisée en ce qu'elle comprend comme matière active un dérivé de cyano-2 imidazopyridine répondant à la formule générale (I)

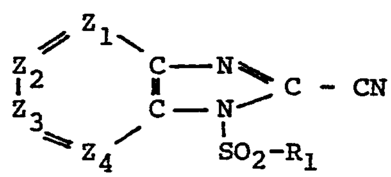

(I)

dans laquelle :

-$R_1$ représente un radical alkyle inférieur éventuellement substitué par un ou plusieurs atomes d'halogène, ou cycloalkyle inférieur éventuellement substitué par un ou plusieurs atomes d'halogène, ou un radical amino, éventuellement substitué par un ou deux radicaux alkyle inférieur, identiques ou différents,

- l'un des groupes $Z_1$, $Z_2$, $Z_3$ et $Z_4$ représente l'atome d'azote et les autres sont choisis indépendemment parmi le groupe CH et les groupes CR dans lesquels R représente un atome d'halogène ou un radical alkyle inférieur éventuellement substitué, alkoxy inférieur éventuellement substitué, alkylthio inférieur éventuellement substitué, nitro ou cyano.

2) - Composition selon la revendication 1 caractérisée en ce que le dérivé de cyano-2 imidazopyridine répond à la formule (I bis) :

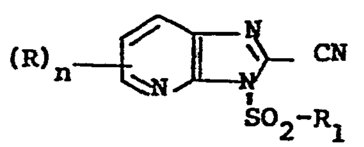

(I bis)

dans laquelle :

- R et $R_1$ ont la même signification que dans la revendication 1 et n est égal à 0, 1 ou 2 étant entendu que lorsque n est égal à 2, les substituants R peuvent être identiques ou différents.

3) - Composition selon la revendication 1 caractérisée en ce que le dérivé de cyano-2 imidazopyridine répond à la formule (I ter) :

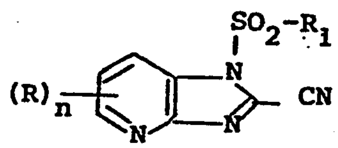

(I ter)

dans laquelle :

- R et $R_1$ ont la même signification que dans la revendication 1 et n est égal à 0, 1 ou 2 étant entendu que lorsque n est égal à 2, les substituants R peuvent être identiques ou différents.

4) - Composition selon l'une des revendications 1 à 3 caractérisée en ce que $R_1$ représente un radical dialkylamino comportant de 2 à 4 atomes de carbone et R représente un atome d'halogène ou un radical alkoxy, alkylthio ou alkyle, éventuellement mono- ou polyhalogéné et comportant de 1 à 3 atomes de carbone, ou le radical nitro ou le radical cyano.

5) - Composition selon l'une des revendications 1 à 4 caractérisée en ce qu'elle comprend de 0,001 % à 95 % en poids de matière active et de 0 à 20 % en poids d'agent tensio-actif.

6) - Procédé de préparation d'un composé répondant à la formule générale (I) de la revendication 1, caractérisé en ce qu'il consiste à faire réagir la cyano-2 imidazopyridine de formule (II)

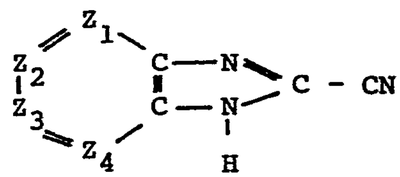

(II)

dans laquelle $Z_1$, $Z_2$, $Z_3$ et $Z_4$, ont la même signification que dans la revendication 1, ou un sel de cette cyano-2 imidazopyridine, sur un halogénure de formule (III) :

$$X - SO_2R_1 \qquad (III)$$

dans laquelle $R_1$ a la même signification que dans la revendication 1 et X représente un atome d'halogène.

7) - Procédé selon la revendication 6 caractérisé en ce que la réaction entre la cyano-2 imidazopyridine et l'halogénure s'effectue dans un solvant aprotique polaire, en présence d'un accepteur d'acide.

8) - Procédé selon la revendication 7 caractérisé en ce que la réaction entre le sel de la cyano-2 imidazopyridine et l'halogénure s'effectue dans un solvant aprotique polaire.

9) - Procédé de traitement des végétaux contre les champignons phytopathogènes caractérisé en ce qu'il consiste à appliquer sur ces végétaux une quantité efficace d'une composition selon l'une des revendications 1 à 5.